(19) Europäisches Patentamt
European Patent Office
Office européen des brevets

(11) Publication number: 0 017 473
A1

(12) EUROPEAN PATENT APPLICATION

(21) Application number: 80301042.0

(22) Date of filing: 02.04.80

(51) Int. Cl.[3]: C 07 D 409/12
A 01 N 47/36, C 07 D 333/42

(30) Priority: 04.04.79 US 27025

(43) Date of publication of application: 15.10.80 Bulletin 80/21

(84) Designated Contracting States: BE CH DE FR GB IT LI LU NL

(71) Applicant: E.I. DU PONT DE NEMOURS AND COMPANY
Legal Department 1007 Market Street
Wilmington, Delaware 19898(US)

(72) Inventor: Levitt, George
3218 Romilly Road
Wilmington Delaware 19810(US)

(74) Representative: Hildyard, Edward Martin et al,
Frank B. Dehn & Co. Imperial House 15-19 Kingsway
London WC2B 6UZ(GB)

(54) Herbicidal thiophenesulfonamides, and preparation, compositions and use thereof.

(57) Compounds of the formula:

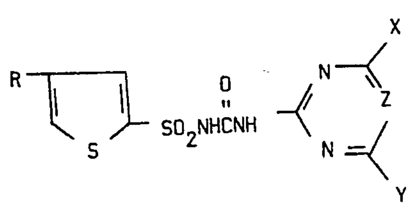

wherein

R is $C_1$-$C_4$ alkyl, $NO_2$, Cl, Br, $SO_2NR_1R_2$ where $R_1$ and $R_2$ are selected from $C_1$-$C_3$ alkyl;

X is H, Cl, Br, $CH_3$, $CH_3CH_2$, alkoxy $C_1$-$C_3$, $CF_3$, $CH_3S$ or $CH_3OCH_2$;

Y is $CH_3$ or $CH_3O$; and

Z is CH or N;

and agriculturally suitable salts thereof, exhibit herbicidal activity especially against broadleaf weeds; they are selective in certain crops, e.g. wheat and barley. The compounds can be formulated for use in conventional manner. They may be made by reacting an appropriately substituted 2-aminotriazine or 2-aminopyrimidine with a substituted thiophenenesulfonyl isocyanate.

EP 0 017 473 A1

## Herbicidal thiophenesulfonamides, and preparation, compositions and use thereof

This invention relates to thiophenesulfonamides and their use as agricultural chemicals and particularly as herbicides.

U.S. Patent 4,127,405 teaches compounds which are useful for controlling weeds in wheat having the formula

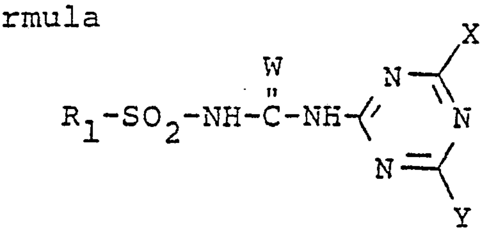

wherein

$R_1$ is

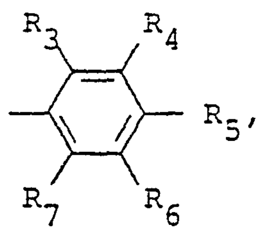, 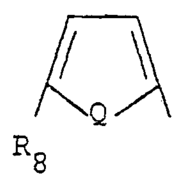, 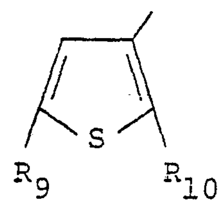

or 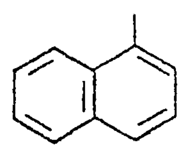 ;

$R_3$ and $R_6$ are independently hydrogen, fluorine, chlorine, bromine, iodine, alkyl of 1-4 carbon atoms, alkoxy of 1-4 carbon atoms, nitro, trifluoromethyl, cyano, $CH_3S(O)_n$- or $CH_3CH_2S(O)_n$-;

$R_4$ is hydrogen, fluorine, chlorine, bromine or methyl;

$R_5$ is hydrogen, fluorine, chlorine, bromine, methyl or methoxy;

$R_7$ is hydrogen, fluorine, chlorine, bromine, alkyl of 1-2 carbon atoms or alkoxy of 1-2 carbon atoms;

$R_8$ is hydrogen, methyl, chlorine or bromine;

$R_9$ and $R_{10}$ are independently hydrogen, methyl, chlorine or bromine;

W and Q are independently oxygen or sulfur;

n is 0, 1 or 2;

X is hydrogen, chlorine, bromine, methyl, ethyl, alkoxy of 1-3 carbon atoms, trifluoromethyl, $CH_3S-$ or $CH_3OCH_2-$; and

Z is methyl or methoxy; or their agriculturally suitable salts; provided that:

(a) when $R_5$ is other than hydrogen, at least one of $R_3$, $R_4$, $R_6$ and $R_7$ is other than hydrogen and at least two of $R_3$, $R_4$, $R_6$ and $R_7$ must be hydrogen;

(b) when $R_5$ is hydrogen and all of $R_3$, $R_4$, $R_6$ and $R_7$ are other than hydrogen, then all of $R_3$, $R_4$, $R_6$ and $R_7$ must be either chlorine or methyl; and

(c) when $R_3$ and $R_7$ are both hydrogen, at least one of $R_4$, $R_5$ or $R_6$ must be hydrogen.

French Patent No. 1,468,747 discloses the following <u>para</u>-substituted phenylsulfonamides, useful as antidiabetic agents:

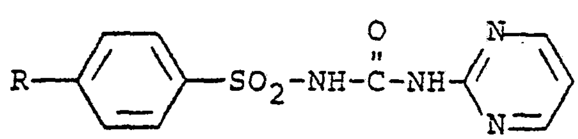

wherein R = H, halogen, $CF_3$ or alkyl.

Logemann et al. Chem. Ab., 53, 18052 g (1959), disclose a number of sulfonamides, including uracil derivatives and those having the formula:

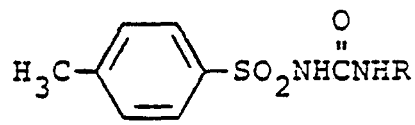

wherein R is butyl, phenyl or 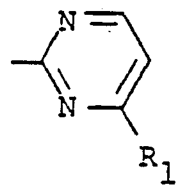 and $R_1$ is hydrogen or methyl. When tested for hypoglycemic effect in rats (oral doses of 25 mg/100 g), the compounds in which R is butyl and phenyl were most potent. The others were of low potency or inactive.

Wojciechowski, J. Acta. Polon. Pharm. 19, p. 121-5 (1962) [Chem Ab., 59 1633 e] describes the synthesis of N-[(2,6-dimethoxypyrimidin-4-yl)aminocarbonyl]-4-methylbenzenesulfonamide:

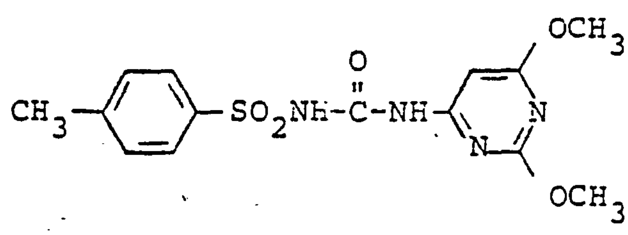

Based upon similarity to a known compound, the author predicted hypoglycemic activity for the foregoing compound.

Netherlands Patent 121,788, published September 15, 1966, teaches the preparation of compounds of Formula (i), and their use as general or selective herbicides,

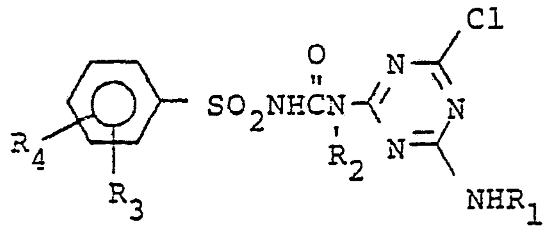

(i)

wherein

$R_1$ and $R_2$ may independently be alkyl of 1-4 carbon atoms; and

$R_3$ and $R_4$ may independently be hydrogen, chlorine or alkyl of 1-4 carbon atoms.

Compounds of Formula (ii), and their use as antidiabetic agents, are reported in J. Drug. Res. 6, 123 (1974),

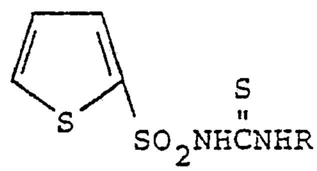

(ii)

wherein R is pyridyl.

The presence of undesired vegetation causes substantial damage to useful crops, especially agricultural products that satisfy man's basic food needs, such as barley, wheat, and the like. The current population explosion and concomitant world food shortage demand improvements in the efficiency of producing these crops. Prevention or minimizing the loss of a portion of such valuable crops by killing, or inhibiting the growth of undesired vegetation is one way of improving this efficiency.

A wide variety of materials useful for killing, or inhibiting (controlling) the growth of undesired

vegetation is available; such materials are commonly referred to as herbicides. The need exists, however, for still more effective herbicides that destroy or retard weeds without causing significant damage to useful crops.

## Summary of the Invention

According to this invention, there are provided compounds of Formula I and their agriculturally suitable salts, suitable agricultural compositions containing them, and methods of using them as selective, as well as general herbicides having both preemergence and postemergence activity or as plant growth regulants. These compounds are highly active herbicides. They are especially useful for controlling broadleaf weeds in wheat and barley.

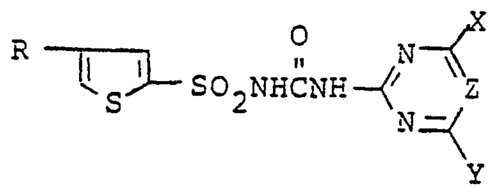

(I)

wherein

R is $C_{1-4}$ alkyl, $NO_2$, Cl, Br, $SO_2NR_1R_2$ where $R_1$ and $R_2$ are selected from $C_1$-$C_3$ alkyl;

X is H, Cl, Br, $CH_3$, $CH_3CH_2$, alkoxy $C_1$-$C_3$, $CF_3$, $CH_3S$ or $CH_3OCH_2$;

Y is $CH_3$ or $CH_3O$; and

Z is CH or N.

Preferred for their higher biological and/or more favorable cost are those compounds of Formula I where:

R is $NO_2$; and/or
X is $CH_3$ or $CH_3O$.

Especially preferred compounds are:

(1) N-[(4-methoxy-6-methyl-2-pyrimidinyl)aminocarbonyl]-4-nitro-2-thiophenesulfonamide; and

(2) N-[(4-methoxy-6-methyl-1,3,5-triazin-2-yl)aminocarbonyl]-4-nitro-2-thiophenesulfonamide.

## Detailed Description

## Synthesis Disclosures

As shown in Equation 1, the compounds of Formula I can be prepared by combining an appropriate 2-aminotriazine or 2-aminopyrimidine of Formula II with an appropriately substituted thiophenesulfonyl isocyanate of Formula III; R, X, Y and Z being as previously defined.

Equation 1

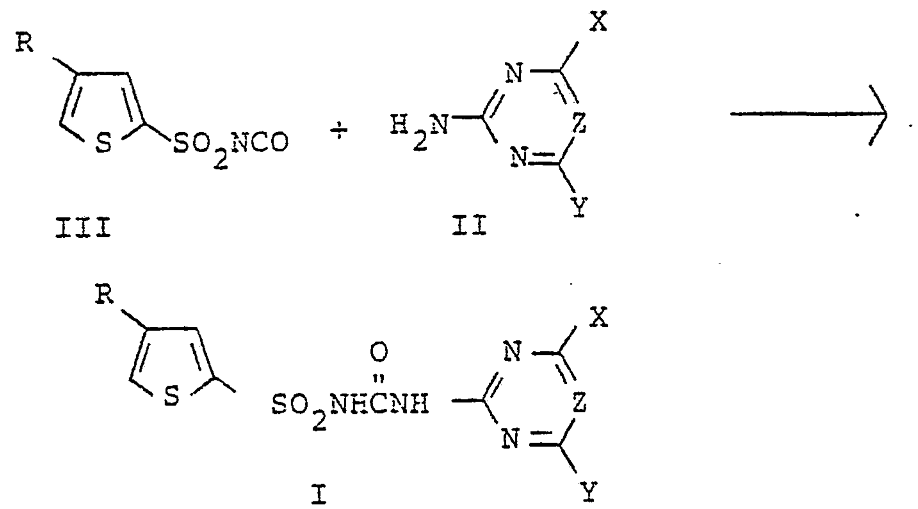

III II I

The reaction is best carried out in inert aprotic solvents such as methylene chloride, acetonitrile or tetrahydrofuran at ambient temperature and pressure. The mode of addition is not critical; however, it is often convenient to add the sulfonyl isocyanate to a

stirred suspension of the aminoheterocycle. Since such isocyanates are usually liquids, or low melting solids soluble in the solvents used, their addition is more easily controlled. Time of reaction is between 1 hour and 24 hours.

The reaction is generally exothermic. In some cases, the desired product crystallizes from the reaction medium in pure form. Products soluble in the reaction mixture are isolated by evaporation of the solvent, trituration of the solid residue with solvents such as 1-chlorobutane, pentane or ethyl ether and filtration.

The intermediate, novel, sulfonyl isocyanates are prepared from the corresponding thiophenesulfonamides via their N-butylcarbamoyl intermediate according to Equations 2 and 3, wherein R is as previously defined.

Equation 2 shows the formation of the intermediate butylcarbamoyl derivative and Equation 3 shows the conversion of this intermediate to the sulfonyl isocyanate by the reaction of phosgene with the N-butyl carbamoyl derivative.

Equation 2

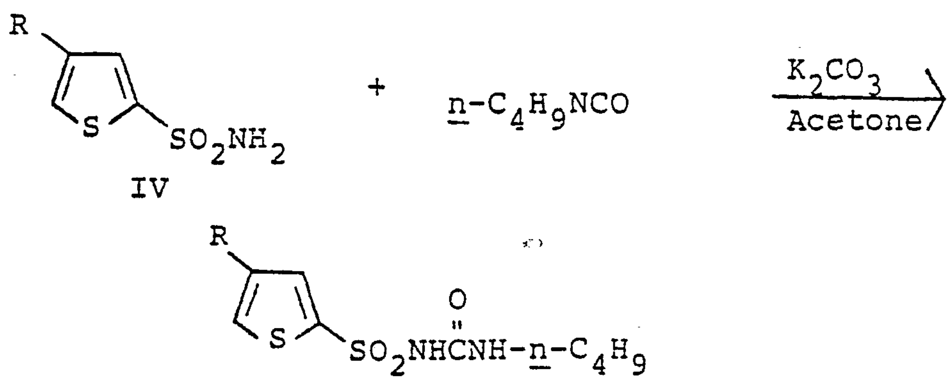


IV

V

Equation 3

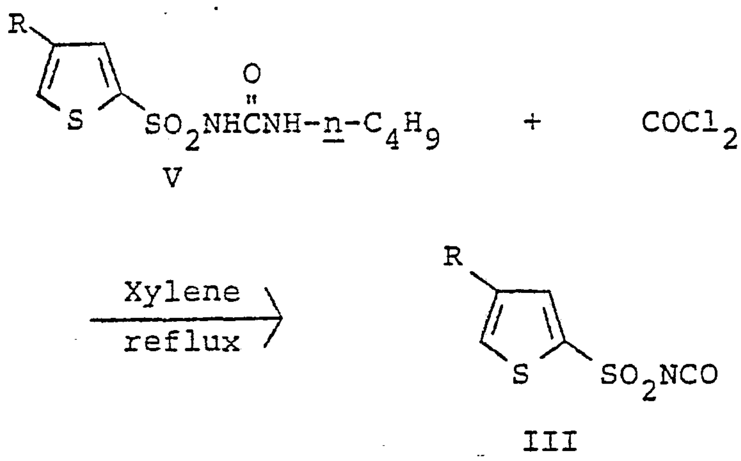


The reaction of Equation 2 is best carried out by combining equivalent amounts of the sulfonamide, butyl isocyanate and potassium carbonate in a polar solvent such as acetone and stirring for 1-16 hours between ambient and reflux temperature. The product is isolated by pouring the mixture into ice-water, adjusting the pH to about 2 and isolating the product (Structure V) by filtration or extraction into methylene chloride.

The product from Equation 2 is dried thoroughly, placed in an aprctic organic solvent such as xylene and heated to reflux under a dry ice cooled condenser. Phosgene is passed into the mixture until an excess is present whereupon the reflux temperature drops to 120° without further addition of phosgene.

Thiophenesulfonyl chlorides and amides can be prepared according to references cited in "Thiophene and its Derivatives", H. D. Hartough, Interscience, New York, 1952. Nitrothiophene sulfonyl chlorides can be prepared according to A. H. Blatt et al., J. Am Chem. Soc. 79, 1693 (1957). The aminotriazines and aminopyrimidines are prepared according to methods

known in the art (see U.S. Patent Specification No. 4,127,405).

Agriculturally suitable salts of the compounds of claim 1 can also be made as described in the above U.S. Patent, to which the reader is referred for further information.

In the following examples, unless otherwise indicated, all parts are by weight and all temperatures in $^{o}C$. Examples 1 and 2 relate to intermediates.

Example 1

N-(Butylaminocarbonyl)-4-nitrothiophene-2-sulfonamide

A stirred suspension of 10 g of 4-nitrothiophene-2-sulfonamide, 6 g of n-butyl isocyanate and 7 g of anhydrous potassium carbonate in 100 ml of acetone was heated to reflux for 3 hours. The resultant viscous mixture was poured into ice-water and the pH adjusted to 2 by the addition of concentrated hydrochloric acid. The resulting tarry precipitate thus obtained was extracted into methylene chloride, separated from the aqueous phase, dried over magnesium sulfate, filtered and concentrated in-vacuo. The residue which showed absorption peaks by nuclear magnetic resonance at .7 to 1.7 multiplet, consistent for butyl and at 8.3 and 8.5, consistent for the thiophene ring was used without further purification in the following reaction.

## Example 2

### 4-Nitrothiophene-2-sulfonyl isocyanate

N-Butylaminocarbonyl-4-nitrothiophenesulfonamide from the preceeding example was stirred in 100 ml of xylene in a round bottom flask equipped with a dry ice condenser, gas inlet tube, thermometer and stirrer. When the temperature of the mixture reached 130° phosgene was passed in until the temperature went down to 120°. The phosgene was turned off until the temperature returned to 130° and the phosgene addition was resumed. This cycling was continued until the reflux temperature would not rise above 120° with the phosgene off, indicating an excess of phosgene in the reaction mixture. After cooling, the mixture was filtered and the filtrate concentrated *in vacuo* to yield 4.8 g of the desired sulfonyl isocyanate as an oil. It showed an infrared absorption peak at 2200 $cm^{-1}$, consistent for the desired sulfonyl isocyanate.

## Example 3

### N-[(4-Methoxy-6-methyl-2-pyrimidinyl)aminocarbonyl]-4-nitrothiophene-2-sulfonamide

To 1.4 g of 2-amino-4-methoxy-6-methylpyrimidine in 25 ml of anhydrous acetonitrile was added 2.4 g of 4-nitrothiophene-2-sulfonyl isocyanate with stirring at ambient temperature. After stirring for sixteen hours, the mixture was filtered and the precipitate washed with 1-chlorobutane. It melted at 155° with decomposition and showed an infrared absorption spectrum with peaks at 1700, 1620 and 1570 $cm^{-1}$ consistent for the desired structure.

By using the procedure of Example 3 with an equivalent amount of the appropriate aminopyrimidine and the appropriately substituted sulfonyl isocyanate, the compounds of Table I can be prepared.

Table I

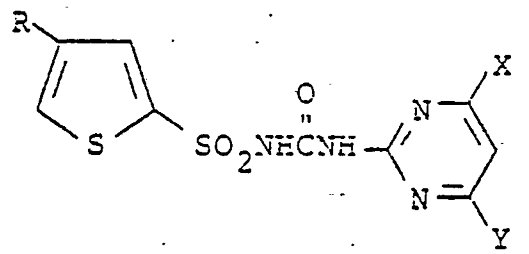

| R | X | Y | m.p.°C |
|---|---|---|---|
| $NO_2$ | $CH_3$ | $CH_3$ | |
| $NO_2$ | $OCH_3$ | $OCH_3$ | |
| Cl | $OCH_3$ | $OCH_3$ | |
| Cl | $CH_3$ | $OCH_3$ | |
| Br | $CH_3$ | $CH_3$ | |
| Br | $CH_3$ | $OCH_3$ | |
| $CH_3$ | $CH_3$ | $OCH_3$ | 167-169° |
| $CH_3$ | $OCH_3$ | $OCH_3$ | |
| $CH_3$ | $OC_2H_5$ | $CH_3$ | |
| $C_2H_5$ | O-n-$C_3H_7$ | $CH_3$ | |
| $CH_3$ | $CF_3$ | $CH_3$ | |
| $CH_2CH_2CH_3$ | $SCH_3$ | $CH_3$ | |
| $CH_3$ | H | $CH_3$ | |
| $CH_3$ | Cl | $CH_3$ | |
| $CH_3$ | Br | $CH_3$ | |
| $CH_2CH_2CH_2CH_3$ | $CH_3OCH_2$ | $CH_3$ | |
| $CH(CH_3)_2$ | $OCH_3$ | $CH_3$ | |
| $SO_2N(CH_3)_2$ | $CH_3$ | $OCH_3$ | |
| 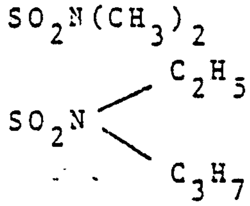 | $CH_3$ | $OCH_3$ | |

Example 4

N-[(4-Methoxy-6-methyl-1,3,5-triazin-2-yl)aminocarbonyl]-4-nitrothiophene-2-sulfonamide

To 1.4 g of 2-amino-4-methoxy-6-methyl-1,3,5-triazine suspended in 25 ml of anhydrous acetonitrile with stirring at ambient temperature was added 2.4 g of 4-nitrothiophene-2-sulfonyl isocyanate. After stirring for sixteen hours at ambient temperature, the product which was present as a precipitate was removed by filtration and washed with 1-chlorobutane. It melted at 188° with decomposition and showed an infrared absorption spectrum peaks at 1740, 1600 and 1560 $cm^{-1}$ consistent for the desired aminocarbonylsulfonamide structure.

By using the procedure of Example 2 with an equivalent amount of the appropriate aminotriazine and the appropriately substituted sulfonyl isocyanate, the compounds of Table II can be prepared.

## Table II

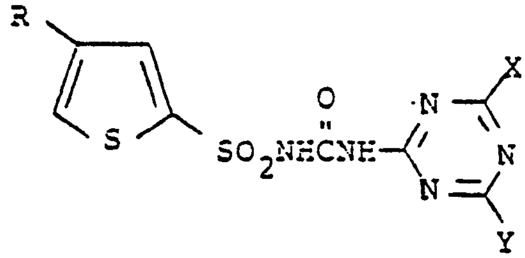

| R | X | Y | m.p.°C |
|---|---|---|---|
| $NO_2$ | $CH_3$ | $CH_3$ | |
| $NO_2$ | $OCH_3$ | $OCH_3$ | |
| $CH_3$ | $CH_3$ | $OCH_3$ | 175-180° |
| $CH_3$ | $CH_3$ | $CH_3$ | |
| $CH_3$ | $OCH_3$ | $OCH_3$ | |
| $C_2H_5$ | $CH_2OCH_3$ | $CH_3$ | |
| $CH(CH_3)_2$ | $CF_3$ | $CH_3$ | |
| $CH_2CH_2CH_2CH_3$ | $OCH_2CH_3$ | $CH_3$ | |
| $CH_3$ | $OCH(CH_3)_2$ | $CH_3$ | |
| $CH_3$ | $SCH_3$ | $OCH_3$ | |
| $CH_3$ | $SCH_3$ | $CH_3$ | |
| Br | H | $CH_3$ | |
| Cl | Cl | $CH_3$ | |
| Cl | Br | $CH_3$ | |
| $SO_2N(CH_3)_2$ | $CH_3$ | $OCH_3$ | |
| 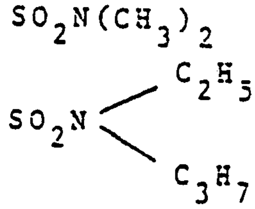 | $CH_3$ | $OCH_3$ | |

Formulations

Useful formulations of the compounds of Formula I can be prepared in conventional ways. They include dusts, granules, pellets, suspensions, emulsions, wettable powders, emulsifiable concentrates and the like. Many of these can be applied directly. Sprayable formulations can be extended in suitable media and used at spray volumes of from a few liters to several hundred liters per hectare. The formulations may, broadly, contain about 0.1% to 99% by weight of active ingredient(s) and at least one of a) about 0.1% to 20% surfactant(s) and b) about 1% to 99.9% solid or liquid diluent(s). More specifically, they will contain these ingredients in the approximate proportions set forth in Table III.

Table III

| | Weight Percent* | | |
|---|---|---|---|
| | Active Ingredient | Diluent(s) | Surfactant(s) |
| Wettable Powders | 20-90 | 0-74 | 1-10 |
| Oil Suspensions, Solutions, Emulsions (including Emulsifiable Concentrates) | 3-50 | 40-95 | 0-15 |
| Aqueous Suspensions | 10-50 | 40-84 | 1-20 |
| Dusts | 1-25 | 70-99 | 0-5 |
| Granules and Pellets | 0.1-95 | 5-99.9 | 0-15 |

*Active Ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation, or by tank mixing.

Some typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide", 2nd Ed., Interscience, New York, 1950. Solubility under 0.1% is preferred for suspension concentrates, solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending, and usually, grinding as in a hammer or fluid energy mill. Suspensions are prepared by wet milling (see for example, Littler, U.S. Patent 3,060,084). Granules and pellets may be made by spraying the active material on preformed granular carriers or by agglomeration techniques. See J. E. Browning, "Agglomeration", Chemical Engineering, Dec. 4, 1967, pp. 147ff. and "Perry's Chemical Engineer's Handbook", 4th Ed., McGraw-Hill, New York, 1963, pp. 8-59ff.

For further information regarding the art of formulation, see for example:

H. M. Loux, U.S. Patent 3,235,361, Col. 6, line 16 through Col. 7, line 19 and Examples 10 through 41.

R. W. Luckenbaugh, U.S. Patent 3,309,192, Col. 5, line 43 through Col. 7, line 62 and Examples 8, 12, 15, 39, 41, 52, 53, 58, 132, 138-140, 162-164, 166, 167, 169-182.

H. Gysin and E. Knusli, U.S. Patent 2,891,855, Col. 3, line 66 through Col. 5, line 17 and Examples 1-4.

G. C. Klingman, "Weed Control as a Science", John Wiley & Sons, Inc., New York, 1961, pp. 81-96.

J. D. Fryer and S. A. Evans, "Weed Control Handbook", 5th Ed., Blackwell Scientific Publications, Oxford, 1968, pp. 101-103.

The reader is referred to the above-cited references for further information regarding the art of formulation.

The disclosures of the above-cited references are herein incorporated by reference.

Unless indicated otherwise, all parts are by weight in the following examples.

Example 5

Wettable Powder

| | |
|---|---|
| N-[(4-Methoxy-6-methyl-2-pyrimidinyl)aminocarbonyl]-4-nitro-2-thiophenesulfonamide | 40% |
| dioctyl sodium sulfosuccinate | 1.5% |
| sodium ligninsulfonate | 3% |
| low viscosity methyl cellulose | 1.5% |
| attapulgite | 54% |

The ingredients are thoroughly blended, passed through an air mill, to produce an average particle size under 15 microns, reblended, and sifted through a U.S.S. No. 50 sieve (0.3 mm opening) before packaging.

Example 6

Granule

| | |
|---|---|
| wettable powder of Example 5 | 10% |
| attapulgite granules (U.S.S. #20-40; 0.84-0.42 mm) | 90% |

A slurry of wettable powder containing 25% solids is sprayed on the surface of attapulgite granules in a double-cone blender. The granules are dried and packaged.

Example 7

Wettable Powder

| | |
|---|---|
| N-[(4-Methoxy-6-methyl-1,3,5-triazin-2-yl)aminocarbonyl]-4-nitro-2-thiophenesulfonamide | 80% |
| sodium alkylnaphthalenesulfonate | 2% |
| sodium ligninsulfonate | 2% |
| synthetic amorphous silica | 3% |
| kaolinite | 13% |

The ingredients are blended and ground in a hammer mill to produce an average particle size under 100 microns. The material is reblended, sifted through a U.S.S. #50 sieve and packaged.

Example 8

Granule

| | |
|---|---|
| wettable powder of Example 7 | 15% |
| gypsum | 69% |
| potassium sulfate | 16% |

The ingredients are blended in a rotating mixer and water sprayed on to accomplish granulation. When most of the material has reached the desired range of 1.0 to 0.42 mm (U.S.S. #18 to 40 sieves), the granules are removed, dried, and screened. Oversize material is crushed to produce additional material in the desired range. These granules contain 12% active ingredient.

Example 9

Wettable Powder

| | |
|---|---|
| N-[(4-Methoxy-6-methyl-2-pyrimidinyl)aminocarbonyl]-4-nitro-2-thiophenesulfonamide | 65% |
| dodecylphenol polyethylene glycol ether | 2% |
| sodium ligninsulfonate | 4% |
| sodium silicoaluminate | 6% |
| montmorillonite (calcined) | 23% |

The ingredients are thoroughly blended. The liquid surfactant is added by spraying upon the solid ingredients in the blender. After grinding in a hammer mill to produce particles essentially all below 100 microns, the material is reblended, sifted through a U.S.S. No. 50 sieve (0.3 mm opening) and packaged.

Example 10

High Strength Concentrate

| | |
|---|---|
| N-[(4-Methoxy-6-methyl-1,3,5-triazin-2-yl)aminocarbonyl]-4-nitro-2-thiophenesulfonamide | 98.5% |
| silica aerogel | 0.5% |
| synthetic amorphous fine silica | 1.0% |

The ingredients are blended and ground in a hammer mill to produce a high strength concentrate essentially all passing a U.S.S. No. 50 sieve (0.3 mm openings). This material may then be formulated in a variety of ways.

Example 11

Aqueous Suspension

| | |
|---|---|
| N-[(4-Methoxy-6-methyl-2-pyrimidinyl)aminocarbonyl]-4-nitro-2-thiophenesulfonamide | 25% |
| hydrated attapulgite | 3% |
| crude calcium ligninsulfonate | 10% |
| sodium dihydrogen phosphate | 0.5% |
| water | 61.5% |

The ingredients are ground together in a ball or roller mill until the solid particles have been reduced to diameters under 10 microns.

Example 12

Oil Suspension

N-[(4-Methoxy-6-methyl-1,3,5-triazin-2-yl)aminocarbonyl]-4-nitro-2-thiophenesulfonamide 25%

| | |
|---|---|
| polyoxyethylene sorbitol hexaoleate | 5% |
| highly aliphatic hydrocarbon oil | 70% |

The ingredients are ground together in a sand mill until the solid particles have been reduced to under about 5 microns. The resulting suspension may be applied directly, but preferably after being extended with oils or emulsified in water.

Example 13

Extruded Pellet

N-[(4-Methoxy-6-methyl-2-pyrimidinyl)aminocarbonyl]-4-nitro-2-thiophenesulfonamide 25%

| | |
|---|---|
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Utility

The compounds of Formula I are useful as herbicides. They may be applied either pre- or postemergence for the control of undesired vegetation in noncrop areas and for selective weed control in certain crops, e.g., wheat and barley. Some of these compounds are useful for the pre- and/or postemergence control of nutsedge. By properly selecting rate and time of application, compounds of this invention may be used to modify plant growth beneficially.

The precise amount of the compound of Formula I to be used in any given situation will vary according to the particular end result desired, the use involved, the weeds to be controlled, the soil type, the formulation and mode of application, weather conditions and like factors. Since so many variables play a role, it is not possible to state a rate of application suitable for all situations. Broadly speaking, the compounds of this invention are used at levels of about 0.01 to 20 kg/ha with a preferred range of 0.05 to 10 kg/ha. The lower rates of the range will generally be selected for lighter soils, for selective weed control in crops or in situations where maximum persistence is not necessary.

The compounds of Formula I may be combined with other herbicides and are particularly useful in combination with 3-(3,4-dichlorophenyl)-1,1-dimethylurea, the triazines such as 2,4-bis(isopropylamino)-6-(methylthio)-s-triazine, the uracils such as 5-bromo-3-sec-butyl-6-methyluracil, N-(phosponomethyl)glycine, 3-cyclohexyl-1-methyl-6-dimethylamino-s-triazine-2,4-(1H,3H)-dione, N,N-dimethyl-2,2-diphenylacetamide, 2,4-dichlorophenoxyacetic acid (and closely related compounds), 4-chloro-2-butynyl-3-chlorophenylcarbamate

(Carbyne®), diisopropylthiolcarbamic acid, ester with 2,3-dichloroallyl alcohol (Avadex®), diisopropylthiolcarbamic acid, S-(2,3,3-trichloroallyl) ester (Avadex® BW), ethyl-N-benzoyl-N-(3,4-dichlorophenyl)-2-aminopropionate (Suffix®), 1,2-dimethyl-3,5-diphenylpyrazoliummethyl-sulfate (Avenge®), methyl 2-[4-(2,4-dichlorophenoxy)phenoxy]-propanoate (Hoelon®), 4-amino-6-tert-butyl-3-(methylthio)-1,2,4-triazin-5(4H)-one (Lexone®), 3-(3,4-dichlorophenyl)-1-methoxy-1-methylurea (Lorox®), 3-isopropyl-1H-2,1,3-benzothiodiazin-(4)-3H-one 2,2-dioxide,α,α,α-trifluoro-2,6-dinitro-N,N-dipropyl-p-toluidine, 1,1'-dimethyl-4,4'bipyridinium ion, monosodium methanearsonate, 2-chloro-2',6',-diethyl-(methoxymethyl)acetanilide, and 1,1-dimethyl-3-(α,α,α-trifluoro-m-tolyl)urea (Cotoran®).

The activity of these compounds was discovered in greenhouse tests. The tests are described and the data resulting from them are shown below.

Test I

Test Procedure

Seeds of crabgrass (Digitaria sp.), barnyardgrass (Echinochloa crusgalli), wild oats (Avena fatua), Cassia tora, morningglory (Ipomoea sp.), cocklebur (Xanthium sp.), sorghum, corn, soybean, rice, wheat and nutsedge tubers were planted in a growth medium and treated preemergence with the chemicals dissolved in a nonphytotoxic solvent. At the same time, cotton having five leaves (including cotyledonary ones), bush beans with the third trifoliolate leaf expanding, crabgrass with two leaves, barnyardgrass with two leaves, wild oats with one leaf, cassia with three leaves (including cotyledonary ones), morningglory with four leaves (including the cotyledonary ones), cocklebur with four leaves (including the cotyledonary ones), sorghum with three leaves, corn with three

leaves, soybean with two cotyledonary leaves, rice with two leaves, wheat with one leaf, and nutsedge with three-five leaves were sprayed. Treated plants and controls were maintained in a greenhouse for 16 days, then all species were compared to controls and visually rated for response to treatment.

Ratings for compounds tested by this procedure are recorded in Table V.

0 = no effect
& or 10 = maximum effect
C = chlorosis or necrosis
D = defoliation
G = growth retardation
H = formative effects
6Y = abscised buds or flowers

Table V

| | | | |
|---|---|---|---|
| | 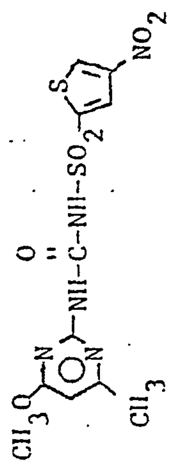 | 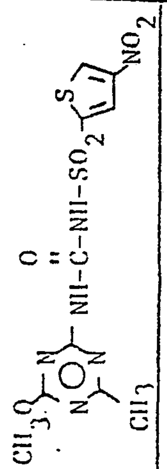 | |
| kg/ha | 0.4 | 0.4 | |
| POST EMERGENCE | | | |
| BUSH BEAN | 8C,8G | 6C,9G | |
| COTTON | 5C,9G | 4C,9G | |
| MORNING GLORY | 5C,9G | 2H,7G | |
| COCKLEBUR | 9C | 9C | |
| CASSIA | 5C,8G | 4C,7G | |
| NUTSEDGE | 2C,7G | 0 | |
| CRABGRASS | 6G | 4G | |
| BARNYARD GRASS | 9H | 2C,7H | |
| WILD OATS | 3C,8G | 0 | |
| WHEAT | 2C,8H | 3G | |
| CORN | 3C,9G | 2C,9G | |
| SOYBEAN | 9C | 5C,9G | |
| RICE | 6C,8G | 4C,9G | |
| SORGHUM | 2C,9G | 5G | |
| PRE EMERGENCE | | | |
| MORNING GLORY | 9G | 9G | |
| COCKLEBUR | 9G | 8G | |
| CASSIA | 9G | 2C,9G | |
| NUTSEDGE | 8G | 0 | |
| CRABGRASS | 1H | 6G | |
| BARNYARD GRASS | 2C,7G | 9H | |
| WILD OATS | 8G | 0 | |
| WHEAT | 8G | 0 | |
| CORN | 9G | 9H | |
| SOYBEAN | 9H | 7H | |
| RICE | 9H | 9H | |
| SORGHUM | 2C,9G | 7G | |

Test II

Wheat and Barley Herbicide Test Procedure

Two ten-inch in diameter plastic pans lined with polyethylene liners were filled with prepared Fallsington silt loam soil. One pan was planted with seeds of wheat (Triticum aestivum), barley (Hordeum vulgare), wild oats (Avena fatua), downy brome (Bromus tectorum), cheatgrass (Bromus secalinus), blackgrass (Alopecurus myosuroides), annual bluegrass (Poa annua), green foxtail (Setaria viridis), quackgrass (Agropyron repens), Italian ryegrass (Lolium multiflorum) and ripgut brome (Bromus rigidus). The other pan was planted with seeds of wild buckwheat (Polygonum convolvulus), Kochia scoparia, smartweed (Polygonum pennsylvanicum), false chamomile (Matricaria inodora), jimsonhill mustard (Sisymbrium altissium), wild mustard (Brassica kaber), tansy mustard (Descurainia pinnata), pigweed (Amaranthus retroflexus) and Russian thistle (Salsola Kali). The above two pans were treated preemergence. At the same time, two pans in which the above plant species were growing were treated postemergence. Plant height at the time of treatment ranged from 1-15 cm depending on plant species.

The compounds applied were diluted with a nonphytotoxic solvent and sprayed over-the-top of the pans. An untreated control and a solvent alone control were included for comparison. All treatments were maintained in the greenhouse for 20 days at which time the treatments were compared to the controls and the effects visually rated. The recorded data are presented in Table VI.

Table VI

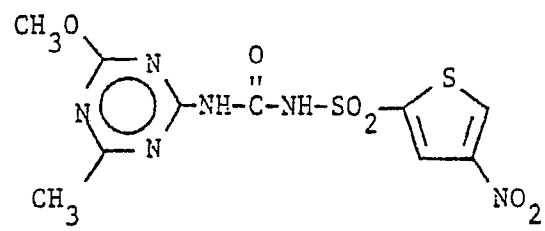

| Rate kg/ha | 0.015 | 0.06 | 0.12 | 0.5 |
|---|---|---|---|---|
| PREEMERGENCE | | | | |
| | | | | |
| wheat | 0 | 0 | 0 | 0 |
| barley | 0 | 0 | 0 | 0 |
| wild oats | 0 | 0 | 0 | 0 |
| downy brome | 0 | 2G | 0 | 0 |
| cheatgrass | 0 | 1G | 0 | 0 |
| blackgrass | 0 | 1G | 0 | 0 |
| annual bluegrass | 0 | 1G | 0 | 0 |
| green foxtail | 0 | 2G | 0 | 0 |
| quackgrass | 0 | 0 | 0 | 0 |
| Italian ryegrass | 0 | 0 | 0 | 0 |
| ripgut brome | 0 | 0 | 0 | 0 |
| Russian thistle | 0 | 2G | 5G | 7C,5G |
| tansy mustard | 0 | 2G | 2G | 9C |
| smartweed | 0 | 3G | 7G | 9C |
| jimhill mustard | 0 | 3G | 2G | 5C,5G |
| Kochia | 0 | 2G | 3C,7G | 7C,8G |
| pigweed | 0 | 4G | 7G | 10C |
| false chamomile | 0 | 2G | 3G | 5C,7G |
| wild mustard | 0 | 2G | 7G | 9C |
| wild buckwheat | 0 | 1G | 3C,5G | 5C,7G |

Table VI (continued)

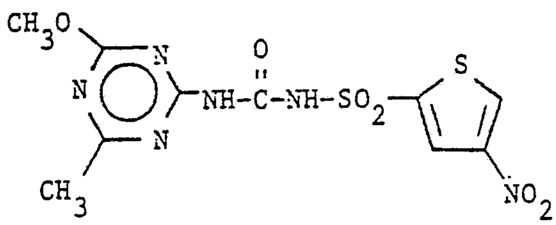

| Rate kg/ha | 0.015 | 0.06 | 0.12 | 0.5 |
|---|---|---|---|---|
| POSTEMERGENCE | | | | |
| | | | | |
| wheat | 0 | 0 | 0 | 2C |
| barley | 0 | 0 | 0 | 1C |
| wild oats | 0 | 0 | 0 | 0 |
| downy brome | 0 | 0 | 0 | 3G |
| cheatgrass | 0 | 0 | 1G | 2C |
| blackgrass | 0 | 0 | 0 | 3C |
| annual bluegrass | 0 | 0 | 0 | 1C |
| green foxtail | 0 | 0 | 1C,2G | 2C |
| quackgrass | 0 | 0 | 2G | 3G |
| Italian ryegrass | 0 | 0 | 0 | 3C |
| ripgut brome | 0 | 0 | 0 | 0 |
| Russian thistle | 10C | 10C | 10C | 10C |
| tansy mustard | 9C | 9C | 10C | 10C |
| smartweed | 5C,5G | 7C | 10C | 10C |
| jimhill mustard | 7C | 10C | 10C | 10C |
| Kochia | 3C | 9C | 9C | 10C |
| pigweed | 9C | 9C | 10C | 10C |
| false chamomile | 2C,3G | 7C | 10C | 10C |
| wild mustard | 9C | 9C | 10C | 10C |
| wild buckwheat | 3C,5G | 8C | 10C | 10C |

Claims:

1. A compound of the formula:

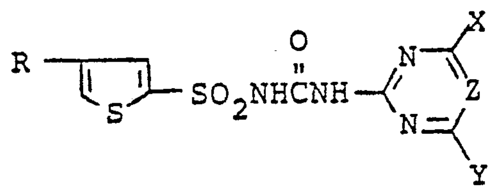

wherein

R is $C_1$-$C_4$ alkyl, $NO_2$, Cl, Br, $SO_2NR_1R_2$ where $R_1$ and $R_2$ are selected from $C_1$-$C_3$ alkyl;

X is H, Cl, Br, $CH_3$, $CH_3CH_2$, alkoxy $C_1$-$C_3$, $CF_3$, $CH_3S$ or $CH_3OCH_2$;

Y is $CH_3$ or $CH_3O$; and

Z is CH or N;

and agriculturally suitable salts thereof.

2. A compound of Claim 1 wherein R is $NO_2$.

3. A compound of Claim 1 wherein X is $CH_3$ or $CH_3O$.

4. A compound of Claim 1 wherein R is $NO_2$ and X is $CH_3$ or $CH_3O$.

5. N-[(4-Methoxy-6-methyl-2-pyrimidinyl)aminocarbonyl]-4-nitro-2-thiophenesulfonamide, and agricularally suitable salts thereof.

6. N-[(4-Methoxy-6-methyl-1,3,5-triazin-2-yl)-aminocarbonyl]-4-nitro-2-thiophensulfonamide, and agriculturally suitable salts thereof.

7. A composition for the control of undesirable vegetation comprising a herbicidal compound and at least one of (a) a surface-active agent and (b) a solid or liquid diluent, characterised in that said herbicidal compound comprises a compound of any of claims 1-6.

8. A method for the control of undesirable vegetation by applying to the locus of such undesirable vegetation an effective amount of a herbicidal compound, characterised in

that said herbicidal compound comprises a compound of any of claims 1-6.

9. A method according to claim 8 wherein said undesirable vegetation is selectively controlled in wheat.

10. A method according to claim 8 wherein said undesirable vegetation is selectively controlled in barley.

11. A method for regulating the growth of vegetation by applying thereto an effective amount of a plant growth regulant characterised in

that said plant growth regulant comprises a compound of any of claims 1-6.

12. A method for making a compound of claim 1 which comprises reacting a 2-aminotriazine or 2-aminopyrimidine of the general formula

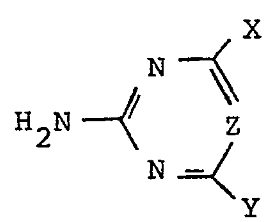

with a 4-substituted thiophenesulfonyl isocyanate of the general formula

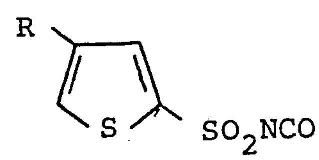

;

R, X, Y and Z being as defined in claim 1.

0017473

Application number

European Patent Office

## EUROPEAN SEARCH REPORT

EP 80 30 1042

| Category | Citation of document with indication, where appropriate, of relevant passages | Relevant to claim |
|---|---|---|
| D | US - A - 4 127 405 (G. LEVITT)<br>* Claims *<br>-- | 1,7-12 |
| P | EP - A - 1 514 (DU PONT DE NEMOURS)<br>* Claims *<br>---- | 1,7-12 |

**DOCUMENTS CONSIDERED TO BE RELEVANT**

CLASSIFICATION OF THE APPLICATION (Int. Cl. 3)

C 07 D 409/12
A 01 N 47/36
C 07 D 333/42

TECHNICAL FIELDS SEARCHED (Int.Cl. 3)

C 07 D 409/12
A 01 N 47/36
C 07 D 333/42

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 26-06-1980 | CHOULY |

EPO Form 1503.1 06.78